# EUROPEAN PATENT APPLICATION

(11) **EP 2 151 441 A1**
(43) Date of publication of application: **10.02.2010**
(21) Application number: 08161939.7
(22) Date of filing: 06.08.2008
(51) Int. Cl.: C07D 471/04

(54) **Beta-carbolin-derivates as substrates for an enzyme**

(71) Applicant: Julius-Maximilians-Universität Würzburg, 97070 Würzburg (DE)
(72) Inventor: Unger, Matthias, 97218, Gerbrunn (DE); Frank, Andreas, 97252, Frickenhausen (DE)
(74) Representative: Ehnis, Tobias

(57) **Abstract**

The invention concerns a first compound of formula 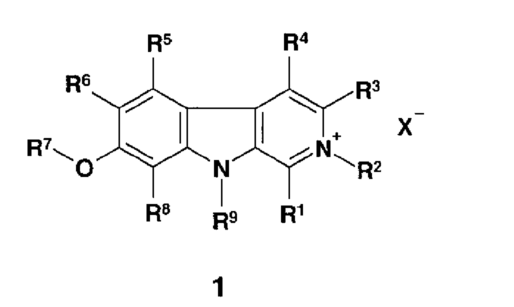 or

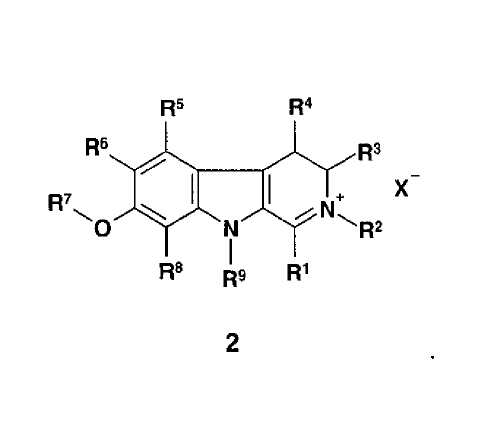

## Description

The present invention concerns new substrates for an enzyme especially an enzyme the substrate of which is a xenobiotic such as a drug or a pesticide. Xenobiotics are substances that are foreign for the human or animal body. Usually, these substances are metabolized in the human or animal body. Especially in the small intestine and the liver there are enzyme systems that modify the xenobiotics such that they become more water soluble. Drugs often lose their pharmacological effect during this modification. Due to the higher solubility in water the drugs lose their ability to enrich in the body and they are excreted quickly via the kidney.

The enzyme systems usually work in two phases. In phase I the xenobiotics are functionalized, i. e., they are provided with a reactive chemical group. In phase II this chemical group is coupled with a strongly water soluble substance such as glucuronic acid or sulfuric acid. The most important enzymes of the phase I metabolism are the cytochrome P450 enzymes (CYP enzymes) that are involved in the oxidation of more than 90 % of all therapeutically important drugs.

The activity of the enzymes that are involved in phase I metabolism is of major importance for the plasma concentration of xenobiotics. Drugs or other substances may inhibit or induce the activity of these enzymes. Practically very important is the influence of drugs on the degradation of other drugs by an inhibition or induction of CYP enzymes. The inhibition of the metabolism of drugs with a narrow therapeutical range such as cyclosporine and phenprocoumon may have serious side effects.

Known strong inhibitors of CYP enzymes are the azole antimycotics ketoconazole and itraconazole as well as the macrolide antibiotics clarithromycin and erythromycin. Substances which shall be used such that they can be taken up by the human or animal body and which inhibit enzymes that are involved in the metabolism of xenobiotics may seriously influence the effect of the xenobiotics. Therefore, it is important to examine the inhibitory activity of the substances on these enzymes. The substances may be drugs, plant extracts, secondary plant metabolites, or pesticides.

From Unger M. and Frank, A., Rapid Commun. Mass Spectrom. 2004, 18, pages 2273-2281 a simultaneous determination of the inhibitory potency of herbal extracts on the activity of six major cytochrome P450 enzymes using liquid chromatography/mass spectrometry and automated online extraction is known. For the determination of the inhibitory activity substrates were incubated with a commercially available mixture of CYP1A2/2C8/2C9/2C19/2D6 and 3A4 and the resulting metabolites were quantified with liquid chromatography and mass spectrometry.

From US 6,420,131 B1 novel fluorescent substrates of human cytochromes P450 enzymes are known. These substrates are useful in assessing cytochrome P450 enzyme activity and in selecting compounds which inhibit cytochrome P450 enzyme activity and, in particular, for identifying potential adverse drug interactions which are mediated by inhibition of cytochrome P450 enzyme activity.

From Takasu, K. et al., Bioorganic and Medicinal Chemistry Letters 14 (2004), pages 1689-1692 and Takasu, K. et al., Chem. Pharm. Bull. (2005), 53(6), pages 653-661 Π-delocalized β-carbolinium cations as potential antimalarials are known.

An object of the present invention is to provide a substrate for an enzyme, a use of a substrate for an enzyme, and a method for synthesizing this substrate. The reaction product of the reaction of the enzyme and the substrate shall be detectable by its fluorescence or its mass.

The object of the present invention is solved by the subject matter of claims 1, 2 and 17. Embodiments are subject matter of claims 3 to 16 and 18 to 23.

According to the invention a first compound of formula 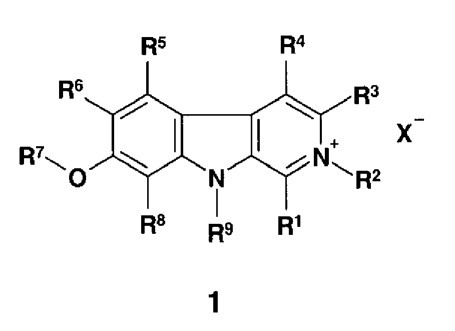 or 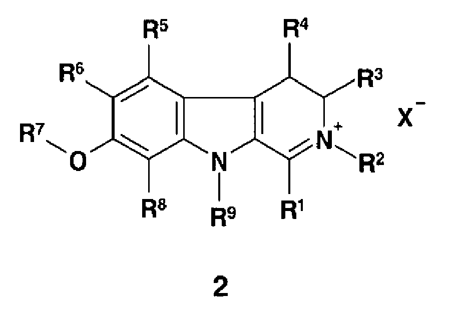 is provided, wherein
R¹, R³ to R⁶, R⁸ and R⁹ represent independently for each occurrence -H, C₁-C₁₈-alkyl, C₂-C₁₈-alkenyl, an aryl residue, a heteroaryl residue, a carboxyl group or an ester of a carboxyl group, a benzyl group, a carboxybenzyl group, an amide group, an acyl group, an alkoxy substituent, an alkyl group bound via a sulfur atom, a sulfonyl group, a nitrogen substituent, a nitrogen substituent bound via a sulfur atom, a hydroxyl group, fluorine, chlorine, bromine, iodine, -CN, -CF₃ or -(CH₂)ₙ-O-R'', wherein n = 0--18 and R'' represents C₁-C₁₈-alkyl, C₂-C₁₈-alkenyl, benzyl, an aryl or a heteroaryl residue,
R² represents C₁-C₁₈-alkyl, C₂-C₁₈-alkenyl, an aryl residue, a heteroaryl residue, a sulfonyl group, a benzyl group, a carboxybenzyl group, or -(CH₂)ₙ-O-R'', wherein n = 0-18 and R'' represents C₁-C₁₈-alkyl, C₂-C₁₈-alkenyl, benzyl, an aryl or a heteroaryl residue, and
R⁷ represents -H, C₁-C₁₈-alkyl, C₂-C₁₈-alkenyl, an aryl residue, a heteroaryl residue, a benzyl group, a carboxybenzyl group, an acyl group, or -(CH₂)ₙ-O-R'', wherein n = 0-18 and R'' represents C₁-C₁₈-alkyl, C₂-C₁₈-alkenyl, benzyl, an aryl or a heteroaryl residue,
wherein X⁻ is a counterion,
wherein the compound of formula 1, in which R¹ and R⁷ = -CH₃, R² = -CH₃, and R³-R⁶, R⁸ and R⁹ = -H, or in which R¹ and R⁷ = - CH₃, R² = -CH₃, R³-R⁶, R⁸ = -H and R⁹ = -CH₃, or in which R¹ and R⁷ = -CH₃, R² = -C₂H₅, R³-R⁶, R⁸ = -H and R⁹ = -CH₃, is excluded.

Independently for each occurrence all alkyl and alkenyl groups of the first compound may be branched, unbranched or cyclic.

The invention further concerns the use of a first compound of formula 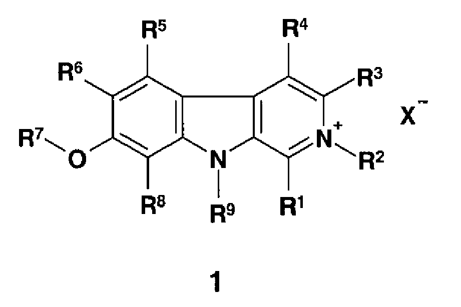 or 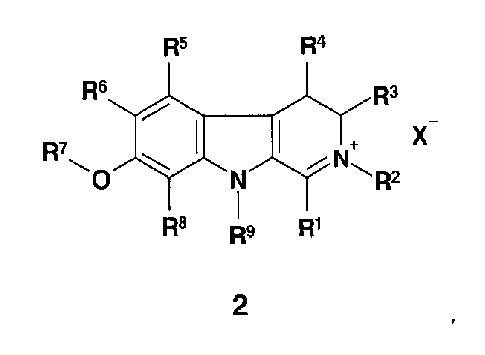 wherein

R¹, R³ to R⁶, R⁸ and R⁹ represent independently for each occurrence -H, C₁-C₁₈-alkyl, C₂-C₁₈-alkenyl, an aryl residue, a heteroaryl residue, a carboxyl group or an ester of a carboxyl group, a benzyl group, a carboxybenzyl group, an amide group, an acyl group, an alkoxy substituent, an alkyl group bound via a sulfur atom, a sulfonyl group, a nitrogen substituent, a nitrogen substituent bound via a sulfur atom, a hydroxyl group, fluorine, chlorine, bromine, iodine, -CN, -CF₃, or - (CH₂)ₙ-O-R'', wherein n = 0-18 and R'' represents C₁-C₁₈-alkyl, C₂-C₁₈-alkenyl, benzyl, an aryl or a heteroaryl residue,
R² represents -H, C₁-C₁₈-alkyl, C₂-C₁₈-alkenyl, an aryl residue, a heteroaryl residue, a sulfonyl group, a benzyl group, a carboxybenzyl group, or -(CH₂)ₙ-O-R'', wherein n = 0-18 and R'' represents C₁-C₁₈-alkyl, C₂-C₁₈-alkenyl, benzyl, an aryl or a heteroaryl residue, and
R⁷ represents -H, C₁-C₁₈-alkyl, C₂-C₁₈-alkenyl, an aryl residue, a heteroaryl residue, a benzyl group, a carboxybenzyl group, an acyl group, or -(CH₂)ₙ-O-R'', wherein n = 0-18 and R'' represents C₁-C₁₈-alkyl, C₂-C₁₈-alkenyl, benzyl, an aryl or a heteroaryl residue,
wherein X⁻ is a counterion
as substrate for an enzyme.

Independently for each occurrence all alkyl and alkenyl groups of the first compound may be branched, unbranched or cyclic. Furthermore, independently for each occurrence the carboxyl group may be -COOR¹⁰, wherein R¹⁰ represents C₁-C₁₈-alkyl, C₂-C₁₈-alkenyl, a benzyl group, an aryl group, or a heteroarylgroup. If the first compound can act as an acid, i.e., if it can undergo a deprotonation, the use also comprises the use of the conjugate base of the first compound.

The enzyme may be an enzyme that usually is involved in the metobolism of xenobiotics, e.g. a drug metabolizing enzyme. The reaction product of this substrate can be detected by its fluorescence or its mass. Fluorescence can be detected in a microtiter plate or after chromatography, especially high performance liquid chromatography (HPLC). It is also possible to detect the mass of the reaction product by mass spectrometry after chromatography, especially HPLC. Thus, the first compound can be used for the in vitro examination of synthetic or herbal drugs or other xenobiotics with respect to their ability to inhibit activity of said enzyme, especially a CYP enzyme. Especially the first compound can be used for the in vitro identification of drug interactions that are due to an inhibition of a CYP enzyme.

The reaction product of the first compound exhibits a strong fluorescence at pH 8 or higher. A positive charge at the nitrogen atom allows a chromatography on a reversed phase column at such a pH. Chromatography enables the separation of the reaction product from substances that interfere with the detection of the reaction product, e. g., due to an own fluorescence or quenching. Especially substances of herbal origin, quench the fluorescence of the reaction product or exhibit an own fluorescence that cannot be separated from the fluorescence of the reaction product. A chromatographical separation of the reaction product from a substance to be tested for its activity allows the detection of the reaction product without the influence of this substance. The chromatographic separation at pH 8 or higher followed by detection of the fluorescence allows a very sensitive detection of the reaction product.

The first compound has a very high affinity to the enzyme, i. e., a low Kₘ value. The Kₘ value that is also designated as Michaelis-Menten constant is the concentration of a substrate of an enzyme at which the half maximal reaction velocity is reached. Furthermore, the first compound is metabolized at a relatively high reaction velocity. Due to the high affinity to the enzyme the first compound can be used as a substrate of the enzyme at a relatively low concentration such that no precipitation of the first compound occurs in the solution in which the enzymatic reaction takes place. Due to the relatively high reaction velocity the reaction product is produced in an amount that is sufficient for a quantitative detection within a reasonable time.

Due to the quaternary nitrogen atom the first compound can also be detected very specifically with high sensitivity by mass spectrometry. Thus, the first compound can be used in different types of assays and with different detection methods with high sensitivity and specificity.

In some embodiments the C₁-C₁₈-alkyl, the C₂-C₁₈-alkenyl, the aryl residue, the heteroaryl residue, and the benzyl group are independently for each occurrence unsubstituted, monosubstituted, or polysubstituted. The aryl residue may be phenyl or naphtyl and the hetero aryl residue may be benzoxazole, benzothiazole, furane, imidazole, oxazole, pyridazin, pyridin, pyrimidin, pyrrol, tetrazole, thiazole, thiophen or triazole. The acyl group can be a formyl group, an acetyl group, a trichloroacetyl group, a fumaryl group, a maleyl group, a succinyl group, a benzoyl group, a branched acyl group or a branched aryl-substituted or heteroaryl-substituted acyl group. The alkoxy substituent may be -O-methyl, -O-ethyl, -O-*n*-propyl, -O-isopropyl, -O-*n*-butyl, -O-isobutyl, -O-*sec*-butyl, or -O-*tert*-butyl. The alkyl group bound via the sulfur atom may be methyl or ethyl. In some embodiments the sulfonyl group is -SO₃H, -SO₂CH₃, -SO₂CF₃, - SO₂C₆H₄CH₃, -SO₂C₆H₄COOH, or -SO₂C₆H₄CH₂Br. The nitrogen substituent may be -NH₂, -NC, -NO₂, -N-CO-R, -NHR, -NRR', and the nitrogen substituent bound via a sulfur atom may be -SO₂NH₂, -SO₂NHR, -SO₂NRR', wherein R and R' represent independently for each occurrence C₁-C₁₈-alkyl, C₂-C₁₈-alkenyl, benzyl, an aryl or a heteroaryl residue.

In some embodiments the substituents R¹, R², R³, R⁴, R⁵, R⁶, R⁷, R⁸, and/or R⁹ are independently for each occurrence halogenated and/or R¹, R², R³, R⁴, R⁵, R⁶, R⁷, R⁸, R⁹, R, R' and/or R'' are independently for each occurrence covalently linked via a further C₁-C₁₈-alkyl, C₂-C₁₈-alkenyl, benzyl, aryl, or heteroaryl residue, wherein none of the C₁-C₁₈-alkyls or C₂-C₁₈ alkenyls is covalently linked via the further C₁-C₁₈-alkyl or C₂-C₁₈ alkenyl. The linkage may be a linkage to one of the C-, N-, or O-atoms of the first compound or of one of the substituents. The halogenated substituents may be represented by - CF₃, -CH₂-CH₂-Cl, or -C₆F₅ and the covalently linked substituents including their linkages may be represented by -CH₂-C₆H₄-COOH, or -(CH₂)₃-O-CH₂-CH₃.

The counterion may be iodide, bromide, chloride, trifluoroacetate, perchlorate, tetrafluoroborate, sulfate or phosphate.

In certain embodiments
R² = benzyl, R⁷ = -CH₃ or -C₂H₅, R¹ = -CH₃ , R³-R⁶ and R⁸ = -H, and R⁹ = -H, -CH₃, -C₂H₅ or benzyl, or
R² = -CH₃, -C₂H₅, or benzyl, R⁷ = benzyl, R¹ = -CH₃, R³-R⁶ and R⁸ = -H, and R⁹ = -H, -CH₃, -C₂H₅, or benzyl, or
R² and R⁷ = -C₂H₅, R¹ = -CH₃, R³-R⁶ and R⁸ = -H, and R⁹ = -H, - CH₃, -C₂H₅, or benzyl, or
R² = -CH₃, R⁷ = -C₂H₅, R¹ = -CH₃, R³-R⁶ and R⁸ = -H, and R⁹ =-H, -CH₃, -C₂H₅, or benzyl, or
R² = -C₂H₅, R⁷ = -CH₃, R¹ = -CH₃, R³-R⁶ and R⁸ = -H, and R⁹ = - H, -CH₃, -C₂H₅, or benzyl, or
R² = -CH₃, -C₂H₅, or benzyl, R⁷ = -C₅H₁₁, R¹ = -CH₃, R³-R⁶ and R⁸ = -H, and R⁹ = -H, -CH₃, -C₂H₅, or benzyl, or
R² and R⁷ = -CH₃, R¹ = -CH₃, R³-R⁶ and R⁸ = -H, and R⁹ = -H, - CH₃, -C₂H₅, or benzyl, or
R² = -H, -CH₃, or -C₂H₅, R¹ and R⁷ = -CH₃, R⁹ = 3-carboxybenzyl or 4-carboxybenzyl, and R³-R⁶ and R³ = -H.

A dimer may be formed from two compounds of formula 1, two compounds of formula 2, or one compound of formula 1 and one compound of formula 2, wherein both compounds are linked via a - -(CH₂)ₙ- group, wherein n = 0 - 18, wherein the -(CH₂)ₙ-group replaces in both compounds independently for each compound one of the residues R¹ to R⁹.

The use of the first compound as a substrate for an enzyme may be for testing a test compound for its inhibitory activity on the enzyme. The use can comprise the following steps:
a) Incubating the first compound, the enzyme and if necessary a coenzyme for the reaction of the enzyme and the first compound in the presence and the absence of the test compound and
b1) performing a first measurement of a fluorescence of the reaction product of the first compound generated in presence of the test compound and performing a second measurement of a fluorescence of the reaction product of the first compound generated in absence of the test compound,
   or
b2) performing a first measurement of a mass of the reaction product of the first compound generated in presence of the test compound and performing a second measurement of a mass of the reaction product of the first compound generated in absence of the test compound,
   and
c) comparing the results of the first measurement and the second measurement.

The enzyme may be a human enzyme. In certain embodiments the enzyme is a cytochrome, especially a cytochrome P450, especially a cytochrome P450 1A2 (CYP1A2), 1A1 (CYP1A1), 2A6 (CYP2A6), 1B1 (CYP1B1), 2B6 (CYP2B6), 2C8 (CYP2C8), 2C9 (CYP2C9), 2C19 (CYP2C19), 2D6 (CYP2D6), 2J2 (CYP2J2), 2E1 (CYP2E1) or 3A4 (CYP3A4) and 4F2 (CYP4F2).

A chromatography may be performed between steps a) and b1) or steps a) and b2). In case of a test compound to be tested which influences the first measurement, e. g., because it has an own fluorescence in the same range of wavelength as the reaction product of the first compound, the chromatography is necessary to enable the first measurement or it simplifies the first measurement and/or it makes it more accurate.

The invention also concerns a method for synthesizing the first compound, wherein a second compound for formula 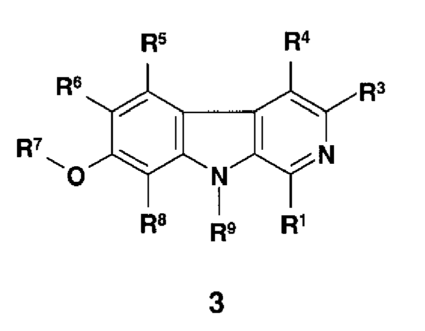 or 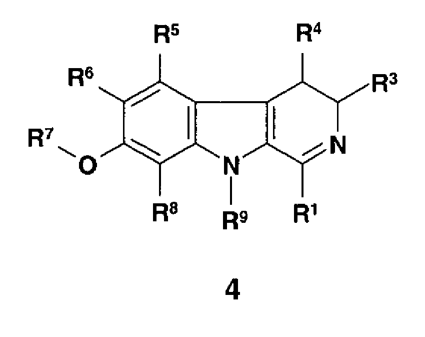 is reacted with an alkylating reagent or a benzylation reagent in a solvent.

In some embodiments the second compound is reacted with the alkylating reagent or the benzylation reagent in the presence of a base. If the second compound is reacted with the alkylating reagent or the benzylation reagent in the presence of a base the first compound is generated as a direct reaction product. If it is reacted in the absence of a base an intermediate product is generated with very high yield that can be converted into the first compound easily.

In certain embodiments the second compound of formula 3 is 1-methyl-9*H*-pyrido[3,4-*b*]indole-7-ol (R¹ = -CH₃, R² - R⁹ = -H) or 7-methoxy-1-methyl-9*H*-pyrido[3,4-*b*]indole (R¹, R⁷ = -CH₃, R² - R⁶, R⁸, R⁹ = -H) or a salt of these compounds and the second compound of formula 4 is 1-methyl-4,9-dihydro-3*H-*pyrido[3,4-*b*]indole-7-ol (R¹ = -CH₃, R² - R⁹ = -H) or 7-methoxy-1-methyl-4,9-dihydro-3*H*-pyrido[3,4-*b*]indole (R¹, R⁷ = -CH₃, R² - R⁶, R⁸, R⁹ = -H) or a salt of these compounds.

The benzylation reagent may be benzyl halogenide or carboxybenzyl halogenide, wherein the halogenide may be chloride, bromide, or iodide and the alkylating reagent may be dimethyl sulfate, diethyl sulfate or diazomethane, alkyl halogenide, wherein the halogenide may be chloride, bromide, or iodide, alkyl triflate, alkyl tosylate, alkyl mesylate, or aryl triflate, wherein the aryl may be phenyl or naphtyl.

The base can be diethylamine, triethylamine, sodium carbonate, potassium carbonate, sodium hydroxide or potassium hydroxide. In some embodiments the solvent is tetrahydrofuran, acetone, dimethyl sulfoxide, acetonitrile, chloroform, dichloromethane or dimethyl formamide. The method according to the invention is very effective if the second compound is reacted with the alkylating reagent or the benzylation reagent at a temperature ranging from 0 to 80 °C.

Embodiments of the invention are described in the following.
- Fig. 1: shows a Michaelis-Menten kinetic of the O-dealkylation of a first compound according to the invention as substrate by CYP3A4,
- Fig. 2: shows the catalytic activity of different CYP enzymes with a first compound according to the invention as substrate, and
- Fig. 3: shows the inhibition of the CYP3A4 catalyzed reaction of a first compound according to the invention by ketoconazole.

### Synthesis

Substances of the basic structure **3** such as 1-methyl-9H-pyrido[3,4-b]indole-7-ol and 7-methoxy-1-methyl-9*H-*pyrido[3,4-b]indole or **4** such as 1-methyl-4,9-dihydro-3*H-*pyrido[3,4-*b*]indole-7-ol and 7-methoxy-1-methyl-4,9-dihydro-3*H*-pyrido[3,4-b]indole or the salts of these substances are reacted with an alkylating reagent or benzylation reagent such as benzyl bromide and a base such as potassium carbonate or potassium hydroxide in an appropriate solvent such as tetrahydrofuran, acetone, dimethyl sulfoxide, acetonitrile, or dimethyl formamide at a temperature ranging from 0 to 80 °C. Reaction products are single and multiple alkylated products according to formula **1** and **2** such as 2-benzyl-7-benzyloxy-1-methyl-9*H*-pyrido[3,4-*b*]indole-2-ium which are suitable as substrates for enzymes, e. g., drug metabolizing enzymes such as CYP enzymes.

If 1-methyl-9*H*-pyrido[3,4-*b*]indole-7-ol according to formula **5** is used with benzyl bromide as benzylation reagent and potassium carbonate as base substance **1a** results as reaction product and substance **1b** as by-product. Substance **1b** is also the product of the enzymatic reaction of the substance **1a.** The synthesis is performed as follows:

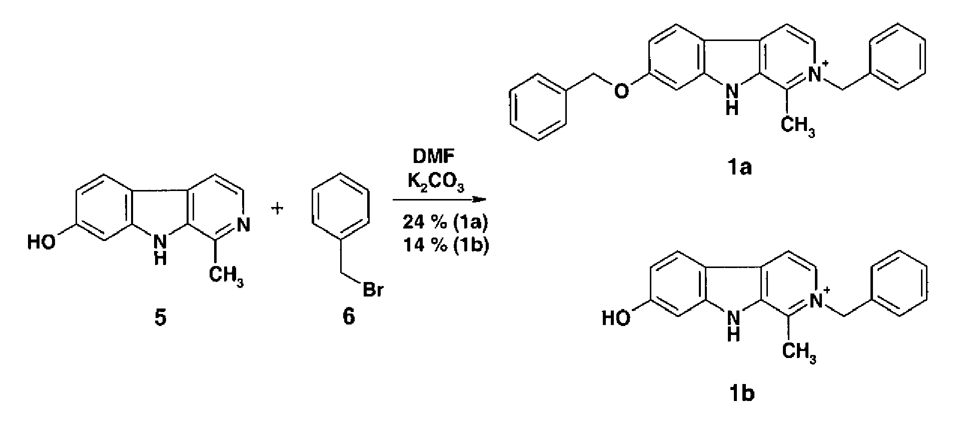

500 mg (2.5 mmol) of 1-methyl-9*H*-pyrido[3,4-b]indole-7-ol **5** were dissolved in 25 ml dimethyl formamide (DMF). 690 mg potassium carbonate (5 mmol) and 0.4 g (2.5 mmol) benzyl bromide **6** were added. After 24 hours of stirring at room temperature the reaction mixture was centrifugated at 3000 g for 10 minutes. The supernatant was collected and 5 ml water were added. Afterwards it was chromatographed on a reversed phase silica gel using acetonitrile, water and 0.05 % trifluoroacetic acid. In this way substances **1a** and **1b** could be separated. After evaporation of the solvent substances **1a** and **1b** remained as white powders. Substances **1a** and **1b** were identified as 2-benzyl-7-benzyloxy-1-methyl-9H-pyrido[3,4-b]indol-2-ium trifluoroacetate salt **1a** and 2-benzyl-7-hydroxy-1-methyl-9H-pyrido[3,4-b]indole-2-ium trifluoroacetate salt **1b.** The counterions are not shown in the above reaction scheme. The yield of substance **1a** as trifluoroacetate salt was 295 mg which corresponds to 0.60 mmol and 24 %. The yield of substance **1b** was 141 mg which corresponds to 0.35 mmol and 14 %.

Substance **1a** can also be synthesized in a two step procedure as follows:
1. Synthesis of the 2-benzyl-7-hydroxy-1-methyl-9H-pyrido[3,4-b]indole-2-ium salt **1b** 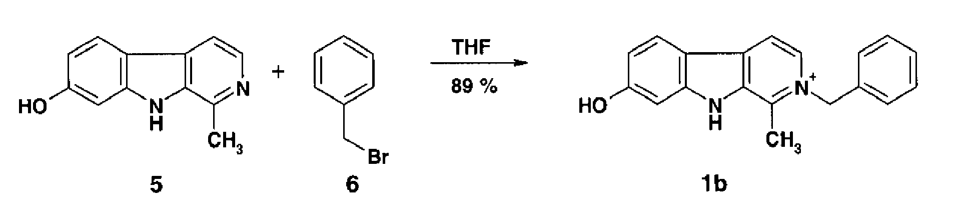 500 mg (2.5 mmol) of 1-methyl-9*H*-pyrido[3,4-b]indole-7-ol **5** were dissolved in 250 ml tetrahydrofuran (THF). 0.4 g (2.5 mmol) benzyl bromide **6** were added. After 24 hours of stirring at 50°C the reaction mixture was stored at 4 °C for 12 hours. Afterwards the precipitate was washed with THF and dissolved in acetonitrile. Afterwards it was chromatographed on a reversed phase 18-silica gel using acetonitrile, water and 0.05 % trifluoroacetic acid. In this way substance **1b** could be separated. After evaporation of the solvent substance **1b** remained as white powder which was identified as 2-benzyl-7-hydroxy-1-methyl-9H-pyrido[3,4-b]indole-2-ium trifluoroacetate salt. The counterion is not shown in the above reaction scheme. The yield of substance **1b** as trifluoroacetate salt was 895 mg which corresponds to 2.23 mmol and 89 %.
2. Synthesis of the 2-benzyl-7-benzyloxy-1-methyl-9*H-*pyrido[3,4-*b*]indole-2-ium salt **1a** 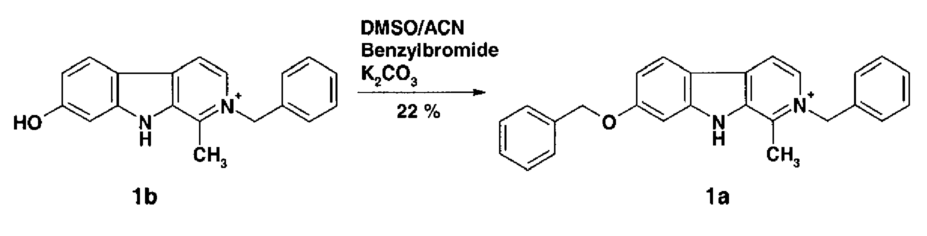 500 mg (1.25 mmol) of the 2-benzyl-7-hydroxy-1-methyl-9H-pyrido[3,4-b]indole-2-ium trifluoroacetate salt **1b** were dissolved in 25 ml of a mixture of 4 parts acetonitrile (ACN) and one part dimethyl sulfoxide (DMSO). 690 mg potassium carbonate (5 mmol) and 0,4 g (2.5 mmol) benzyl bromide were added. After 12 hours of stirring at room temperature the reaction mixture was centrifugated at 3000 g for 10 minutes. The supernatant was collected and 5 ml water were added. Afterwards it was chromatographed on a reversed phase silica gel using acetonitrile, water and 0.05 % trifluoroacetic acid. In this way substance **1a** could be separated. After evaporation of the solvent substance **1a** remained as white powder. Substance **1a** was identified as 2-benzyl-7-benzyloxy-1-methyl-9H-pyrido[3,4-b]indole-2-ium trifluoroacetate salt. The counterion is not shown in the above reaction scheme. The yield of substance **1a** as trifluoroacetate salt was 133 mg which corresponds to 0.27 mmol and 22 %.

### Enzyme Assay

The Michaelis-Menten constant (Kₘ) and the maximal reaction velocity (Vₘₐₓ) are important parameters for inhibition assays. To determine the inhibitory activity of test compounds the substrate concentration in the assay should be about the Kₘ value. For the determination of Kₘ and Vₘₐₓ of the following CYP3A4-catalyzed O-dealkylation reaction of substance **1a** 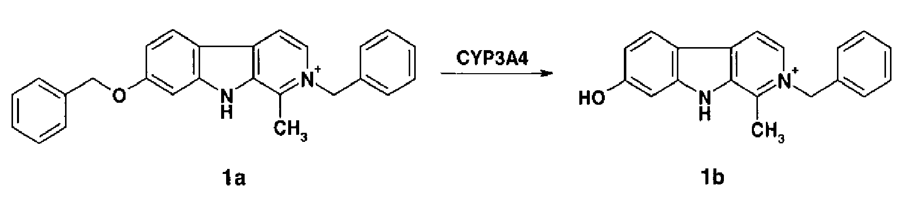 different concentrations (0.1, 0,5, 1, 2.5, 5, 10, 25, 50, 75, 100 µM) of substance **1a** were contacted with 5 pmol/ml of recombinant human CYP3A4 (Supersomes^{®}, BD Biosciences, 2350 Qume Drive, San Jose, CA USA 95131) and 2 mM NADPH as coenzyme in a 100 mM potassium phosphate puffer (pH 7.4; 3 mM MgCl₂). After incubation for 20 minutes at 37°C the reaction product **1b** was quantified using mass spectrometry after liquid chromatography.

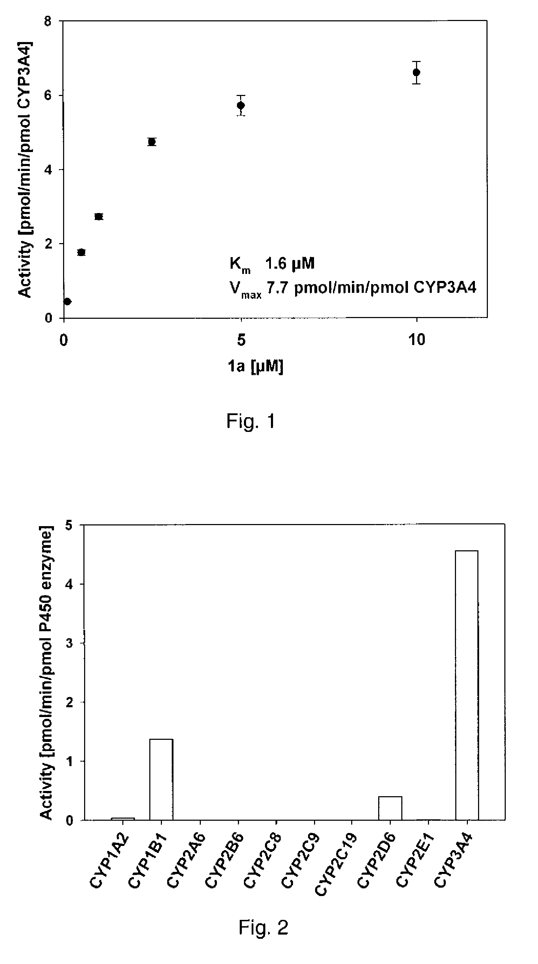

From Fig. 1 it can be seen that the enzymatic reaction of substance **1a** as substrate shows a Michaelis-Menten kinetic in the range from 0.1 to 10 µM whereas a inhibition by the substrate occurs at higher concentrations of the substrate. For the calculation of Kₘ and Vₘₐₓ the Sigma Plot 10.0 software with enzyme kinetic module (SPSS, Inc., Chicago, IL, USA) was used. For substance **1a** the resulting Kₘ value is 1.6 µM and Vₘₐₓ is 7.7 pmol/min/pmol CYP3A4.

Having a Kₘ value of 1.6 µM substance **1a** has an affinity for CYP3A4 that is much higher than that of the usually used fluorescence substrate 7-benzyloxy-4-trifluoromethylcumarin (BFC) and benzyloxyresorufin (BzRes). This can be seen from the following comparison:

| **Substrate** | **Metabolite** | **Kₘ** [µM] | **Vₘₐₓ** [pmol/min/pmol CYP3A4] |
|---|---|---|---|
| **1a** | **1b** | 1.6 | 7.7 |
| **BFC¹** | 7-Hydroxy-4-trifluoromethylcoumarin | >200 | 1.5 (at 40 µM BFC) |
| **BzRes¹** | Resorufin | 38 | 0.3 |
| **Midazolam²** | 1-Hydroxymidazolam | 0.6 | 16.3 |

| | | | |
|---|---|---|---|
| ¹ according to US 6,207,404 ² according to Walsky R.L., Obach R.S., 2004, Drug Metab Dispos 32: 647-660 | | | |

Whereas BFC and BzRes are used in inhibition assays at a concentration of 50 µM substance **1a** can be used in inhibition assays at a 25 fold lower substrate concentration such that no solubility problems occur. Furthermore, reaction velocity of substance **1a** is about 5-fold higher than that of BFC. The low Kₘ value of substance **1a** and its high reaction velocity characterize this substance as an ideal substrate for CYP3A4.

To determine the catalytic activity of CYP3A4 for the reaction with substance **1a** 2 µM of this substance, a concentration that corresponds to the Kₘ value, were contacted with 5 pmol/ml recombinant human CPY3A4 (Supersomes^{®}, BD Biosciences) and 2 mM NADPH in 100 mM potassium phosphate puffer (pH 7.4; 3 mM MgCl₂). After incubation for 20 minutes at 37°C the metabolite **1b** was quantified using liquid chromatography followed by mass spectrometry. For this reaction 4.5 pmol/min/pmol CYP3A4 could be determined as activity of the enzyme. According to US 6,207,404 the activity of CYP3A4 for the reaction of BFC at a concentration of 50 µM is about 1.5 pmol/min/pmol CYP3A4. This shows that the activity of the enzyme for the reaction of substance **1a** is 3-fold higher than that for the reaction of BFC.

For the determination of the catalytic activity of other CYP-enzymes with respect to the reaction of substance **1a** 2 µM of substance **1a** were contacted with 15 pmol/ml CYP enzyme (Supersomes^{®}, BD Biosciences) and 2 mM NADPH in 100 mM potassium phosphate puffer (pH 7.4; 3 mM MgCl₂) with the exception of CYP3A4 that was used at a concentration of 5 pmol/ml. As can be seen from Fig. 2 the reaction of substrate **1a** to reaction product **1b** could be catalyzed by CYP3A4, CYP1B1 and CYP2D6.

For the determination of the IC₅₀ value of the CYP3A4-inhibitor ketoconazole this inhibitor was used in concentrations of 0.00025-10 µM with 2 µM substance **1a,** 5 pmol/ml recombinant human CYP3A4 (Supersomes^{®}, BD Biosciences) and 2 mM NADPH in 100 mM potassium phosphate puffer (pH 7.4; 3 mM MgCl₂). After incubation for 20 minutes at 37°C the metabolite **1b** was quantified using liquid chromatography and mass spectrometry. For the calculation of the IC₅₀ value the Sigma Plot 10.0 software with enzyme kinetic module was used.

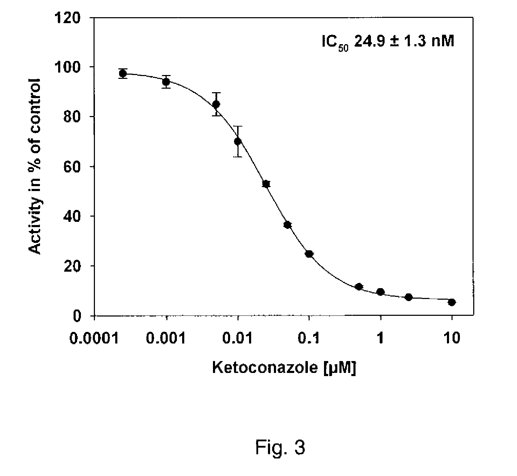

The result of the inhibition can be seen in Fig. 3. The calculated IC₅₀ value is 24.9 ± 1.3 nM. This corresponds to the values that had been determined for CYP3A4 using BFC and midazolam as substrate.

## Claims

1. A first compound of formula 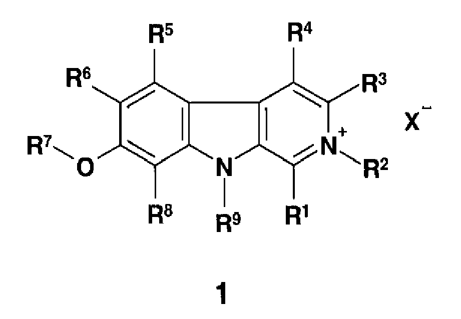 or 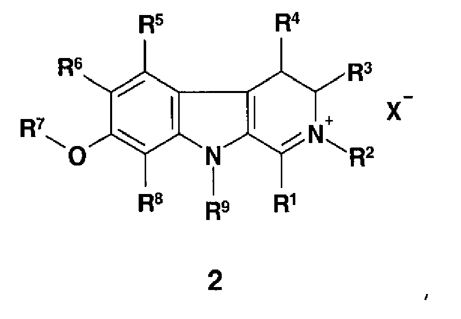 wherein
R¹, R³ to R⁶, R⁸ and R⁹ represent independently for each occurrence -H, C₁-C₁₈-alkyl, C₂-C₁₈-alkenyl, an aryl residue, a heteroaryl residue, a carboxyl group or an ester of a carboxyl group, a benzyl group, a carboxybenzyl group, an amide group, an acyl group, an alkoxy substituent, an alkyl group bound via a sulfur atom, a sulfonyl group, a nitrogen substituent, a nitrogen substituent bound via a sulfur atom, a hydroxyl group, fluorine, chlorine, bromine, iodine, -CN, -CF₃ or -(CH₂)ₙ-O-R'' , wherein n = 0-18 and R'' represents C₁-C₁₈-alkyl, C₂-C₁₈-alkenyl, benzyl, an aryl or a heteroaryl residue,
R² represents C₁-C₁₈-alkyl, C₂-C₁₈-alkenyl, an aryl residue, a heteroaryl residue, a sulfonyl group, a benzyl group, a carboxybenzyl group, or -(CH₂)ₙ-O-R'', wherein n = 0-18 and R'' represents C₁-C₁₈-alkyl, C₂-C₁₈-alkenyl, benzyl, an aryl or a heteroaryl residue, and
R⁷ represents -H, C₁-C₁₈-alkyl, C₂-C₁₈-alkenyl, an aryl residue, a heteroaryl residue, a benzyl group, a carboxybenzyl group, an acyl group, or -(CH₂)ₙ-O-R'', wherein n = 0-18 and R'' represents C₁-C₁₈-alkyl, C₂-C₁₈-alkenyl, benzyl, an aryl or a heteroaryl residue,
wherein X⁻ is a counterion,
wherein the compound of formula 1, in which R¹ and R⁷ = -CH₃, R² = -CH₃, and R³-R⁶, R⁸ and R⁹ = -H, or in which R¹ and R⁷ = - CH₃, R² = -CH₃, R³-R⁶, R⁸ = -H and R⁹ = -CH₃, or in which R¹ and R⁷ = -CH₃, R² = -C₂H₅, R³-R⁶, R⁸ = -H and R⁹ = -CH₃, is excluded.

2. Use of a first compound of formula 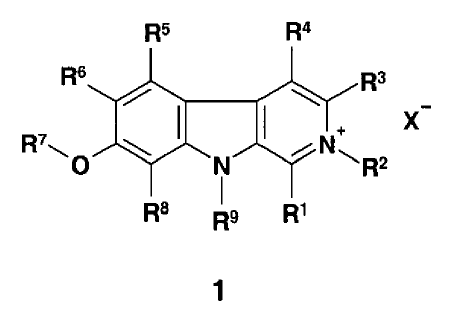 or 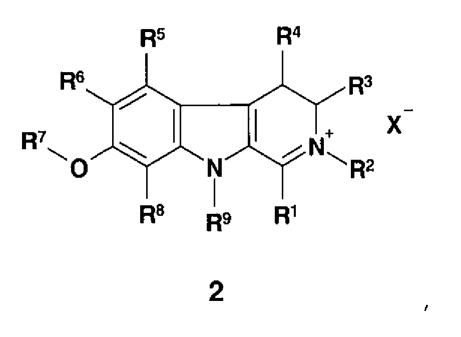 wherein
R¹, R³ to R⁶, R⁸ and R⁹ represent independently for each occurrence -H, C₁-C₁₈-alkyl, C₂-C₁₈-alkenyl, an aryl residue, a heteroaryl residue, a carboxyl group or an ester of a carboxyl group, a benzyl group, a carboxybenzyl group, an amide group, an acyl group, an alkoxy substituent, an alkyl group bound via a sulfur atom, a sulfonyl group, a nitrogen substituent, a nitrogen substituent bound via a sulfur atom, a hydroxyl group, fluorine, chlorine, bromine, iodine, -CN, -CF₃, or - -(CH₂)ₙ-O-R'', wherein n = 0-18 and R'' represents C₁-C₁₈-alkyl, C₂-C₁₈-alkenyl, benzyl, an aryl or a heteroaryl residue,
R² represents -H, C₁-C₁₈-alkyl, C₂-C₁₈-alkenyl, an aryl residue, a heteroaryl residue, a sulfonyl group, a benzyl group, a carboxybenzyl group, or -(CH₂)ₙ-O-R'', wherein n = 0-18 and R'' represents C₁-C₁₈-alkyl, C₂-C₁₈-alkenyl, benzyl, an aryl or a heteroaryl residue, and
R⁷ represents -H, C₁-C₁₈-alkyl, C₂-C₁₈-alkenyl, an aryl residue, a heteroaryl residue, a benzyl group, a carboxybenzyl group, an acyl group, or -(CH₂)ₙ-O-R'', wherein n = 0-18 and R'' represents C₁-C₁₈-alkyl, C₂-C₁₈-alkenyl, benzyl, an aryl or a heteroaryl residue,
wherein X⁻ is a counterion
as substrate for an enzyme.

3. The first compound of claim 1 or the use of claim 2, wherein the C₁-C₁₈-alkyl, the C₂-C₁₈-alkenyl, the aryl residue, the heteroaryl residue, and the benzyl group are independently for each occurrence unsubstituted, monosubstituted, or polysubstituted.

4. The first compound of claim 1 or 3 or the use of claim 2 or 3, wherein the aryl residue is phenyl or naphtyl and the heteroaryl residue is benzoxazole, benzothiazole, furane, imidazole, oxazole, pyridazin, pyridin, pyrimidin, pyrrol, tetrazole, thiazole, thiophen or triazole.

5. The first compound of claim 1, 3 or 4 or the use of one of the claims 2 to 4, wherein the acyl group is a formyl group, an acetyl group, a trichloroacetyl group, a fumaryl group, a maleyl group, a succinyl group, a benzoyl group, a branched acyl group or a branched aryl-substituted or heteroaryl-substituted acyl group.

6. The first compound of one of the claims 1 and 3 to 5 or the use of one of the claims 2 to 5, wherein the alkoxy substituent is -O-methyl, -O-ethyl, -O-*n*-propyl, -O-isopropyl, - O-*n*-butyl, -O-isobutyl, -O-*sec*-butyl, or -O-*tert*-butyl.

7. The first compound of one of the claims 1 and 3 to 6 or the use of one of the claims 2 to 6, wherein the alkyl group bound via the sulfur atom is methyl or ethyl.

8. The first compound of one of the claims 1 and 3 to 7 or the use of one of the claims 2 to 7, wherein the sulfonyl group is -SO₃H, -SO₂CH₃, -SO₂CF₃, -SO₂C₆H₄CH₃, -SO₂C₆H₄COOH, or SO₂C₆H₄CH₂Br.

9. The first compound of one of the claims 1 and 3 to 8 or the use of one of the claims 2 to 8, wherein the nitrogen substituent is -NH₂, -NC, -NO₂, -N-CO-R, -NHR, -NRR', and the nitrogen substituent bound via a sulfur atom is -SO₂NH₂, - SO₂NHR, -SO₂NRR', wherein R and R' represent independently for each occurrence C₁-C₁₈-alkyl, C₂-C₁₈-alkenyl, benzyl, an aryl or a heteroaryl residue.

10. The first compound of one of the claims 1 and 3 to 9 or the use of one of the claims 2 to 9, wherein R¹, R², R³, R⁴, R⁵, R⁶, R⁷, R⁸, and/or R⁹ are independently for each occurrence halogenated and/or wherein R¹, R², R³, R⁴, R⁵, R⁶, R⁷, R⁸, R⁹, R, R' and/or R'' are independently for each occurrence covalently linked via a further C₁-C₁₈-alkyl, C₂-C₁₈-alkenyl, benzyl, aryl, or heteroaryl residue, wherein none of the C₁-C₁₈-alkyls or C₂-C₁₈ alkenyls is covalently linked via the further C₁-C₁₈-alkyl or C₂-C₁₈ alkenyl.

11. The first compound of one of the claims 1 and 3 to 10 or the use of one of the claims 2 to 10, wherein the counterion is iodide, bromide, chloride, trifluoroacetate, perchlorate, tetrafluoroborate, sulfate or phosphate.

12. The first compound of one of the claims 1 and 3 to 11 or the use of one of the claims 2 to 11, wherein
R² = benzyl, R⁷ = -CH₃ or -C₂H₅, R¹ = -CH₃, R³-R⁶ and R⁸ = -H, and R⁹ = -H, -CH₃, -C₂H₅ or benzyl, or
R² = -CH₃, -C₂H₅, or benzyl, R⁷ = benzyl, R¹ = -CH₃, R³-R⁶ and R⁸ = -H, and R⁹ = -H, -CH₃, -C₂H₅, or benzyl, or
R² and R⁷ = -C₂H₅, R¹ = -CH₃, R³-R⁶ and R⁸ = -H, and R⁹ = -H, - CH₃, -C₂H₅, or benzyl, or
R² = -CH₃, R⁷ = -C₂H₅, R¹ = -CH₃, R³-R⁶ and R⁸ = -H, and R⁹ = - H, -CH₃, -C₂H₅, or benzyl, or
R² = -C₂H₅, R⁷ = -CH₃, R¹ = -CH₃, R³-R⁶ and R⁸ = -H, and R⁹ = - H, -CH₃, -C₂H₅, or benzyl, or
R² = -CH₃, -C₂H₅, or benzyl, R⁷ = -C₅H₁₁, R¹ = -CH₃, R³-R⁶ and R⁸ = -H, and R⁹ = -H, -CH₃, -C₂H₅, or benzyl, or
R² and R⁷ = -CH₃, R¹ = -CH₃, R³-R⁶ and R⁸ = -H, and R⁹ = -H, - CH₃, -C₂H₅, or benzyl, or
R² = -H, -CH₃, or -C₂H₅, R¹ and R⁷ = -CH₃, R⁹ = 3-carboxybenzyl or 4-carboxybenzyl, and R³-R⁶ and R⁸ = -H.

13. The first compound of one of the claims 1 and 3 to 12 or the use of one of the claims 2 to 12, wherein a dimer is formed from two compounds of formula 1, two compounds of formula 2, or one compound of formula 1 and one compound of formula 2, wherein both compounds are linked via a -(CH₂)ₙ-group, wherein n = 0 - 18, wherein the -(CH₂)ₙ- group replaces in both compounds independently for each compound one of the residues R¹ to R⁹.

14. The use of one of the claims 2 to 13 for testing a test compound for its inhibitory activity on the enzyme comprising the following steps:
a) Incubating the first compound, the enzyme and if necessary a coenzyme for the reaction of the enzyme and the first compound in the presence and the absence of the test compound and
b1) performing a first measurement of a fluorescence of the reaction product of the first compound generated in presence of the test compound and performing a second measurement of a fluorescence of the reaction product of the first compound generated in absence of the test compound,
or
b2) performing a first measurement of a mass of the reaction product of the first compound generated in presence of the test compound and performing a second measurement of a mass of the reaction product of the first compound generated in absence of the test compound,
and
c) comparing the results of the first measurement and the second measurement.

15. The use of one of the claims 2 to 14, wherein the enzyme is a cytochrome, especially a cytochrome P450, especially a cytochrome P450 1A2 (CYP1A2), 1A1 (CYP1A1), 2A6 (CYP2A6), 1B1 (CYP1B1), 2B6 (CYP2B6), 2C8 (CYP2C8), 2C9 (CYP2C9), 2C19 (CYP2C19), 2D6 (CYP2D6), 2J2 (CYP2J2), 2E1 (CYP2E1) or 3A4 (CYP3A4) and 4F2 (CYP4F2).

16. The use of one of the claims 2 to 15, wherein a chromatography is performed between steps a) and b1) or steps a) and b2).

17. A method for synthesizing the first compound as specified in any of the claims 2 to 13, wherein a second compound of formula 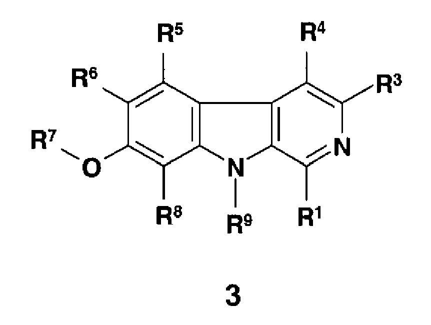 or 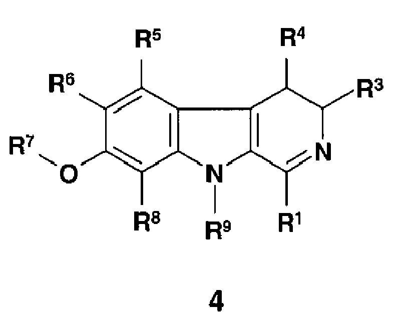 is reacted with an alkylating reagent or a benzylation reagent in a solvent.

18. The method of claim 17, wherein the second compound of formula 3 is 1-methyl-9*H*-pyrido[3,4-*b*]indole-7-ol (R¹ = -CH₃, R² - R⁹ = -H) or 7-methoxy-1-methyl-9*H*-pyrido[3,4-*b*]indole (R¹, R⁷ = -CH₃, R² - R⁶, R⁸, R⁹ = -H) or a salt of these compounds and the second compound of formula 4 is 1-methyl-4,9-dihydro-3*H*-pyrido[3,4-*b*]indole-7-ol (R¹ = -CH₃, R² - R⁹ = -H) or 7-methoxy-1-methyl-4,9-dihydro-3*H*-pyrido[3,4-*b*]indole (R¹, R⁷ = -CH₃, R² - R⁶, R⁸, R⁹ = -H) or a salt of these compounds.

19. The method of claim 17 or 18, wherein the second compound is reacted with the alkylating reagent or the benzylation reagent in the presence of a base.

20. The method of any of claims 17 to 19, wherein the benzylation reagent is benzyl halogenide or carboxybenzyl halogenide, wherein the halogenide may be chloride, bromide, or iodide and the alkylating reagent is dimethyl sulfate, diethyl sulfate or diazomethane, alkyl halogenide, wherein the halogenide may be chloride, bromide, or iodide, alkyl triflate, alkyl tosylate, alkyl mesylate, or aryl triflate, wherein the aryl may be phenyl or naphtyl.

21. The method of any of claims 17 to 20, wherein the base is diethylamine, triethylamine, sodium carbonate, potassium carbonate, sodium hydroxide or potassium hydroxide.

22. The method of any of claims 17 to 21, wherein the solvent is tetrahydrofuran, acetone, dimethyl sulfoxide, acetonitrile, chloroform, dichloromethane or dimethyl formamide.

23. The method of any of claims 17 to 22, wherein the second compound is reacted with the alkylating reagent or the benzylation reagent at a temperature ranging from 0 to 80 °C.
